# EUROPÄISCHE PATENTANMELDUNG

(11) **EP 3 078 945 A1**
(43) Veröffentlichungstag der Anmeldung: **12.10.2016**
(21) Anmeldenummer: 16161952.3
(22) Anmeldetag: 23.03.2016
(51) Int. Cl.: G01H 1/00, G01M 13/04

(54) **VERFAHREN UND VORRICHTUNG ZUR ZUSTANDSÜBERWACHUNG VON MASCHINEN**

(30) Priorität: 09.04.2015 AT 502802015
(71) Anmelder: Hainzl Industriesysteme GmbH, 4020 Linz (AT)
(72) Erfinder: Riegler, Christian Michael, 4020 Linz (AT)
(74) Vertreter: Patentanwaltskanzlei Hübscher

(57) **Zusammenfassung**

Es werden ein Verfahren und eine Vorrichtung zur Zustandsüberwachung von Maschinen (1) mit rotierenden, durch ein hydraulisches Schmiermittel geschmierten Bauteilen (2) beschrieben, wobei von den rotierenden Bauteilen (2) erregte Schwingungen sowie zumindest ein zusätzlicher vom Betriebszustand der Maschinen (1) abhängiger Parameter erfasst und in Abhängigkeit von diesem zusätzlich erfassten Parameter mit einem Schwellwert verglichen werden. Um vorteilhafte Überwachungsbedingungen zu schaffen, wird vorgeschlagen, dass als zusätzlicher Parameter der Feststoffanteil des hydraulischen Schmiermittels erfasst wird.

## Beschreibung

Die Erfindung bezieht sich auf ein Verfahren zur Zustandsüberwachung von Maschinen mit rotierenden, durch ein hydraulisches Schmiermittel geschmierten Bauteilen, wobei von den rotierenden Bauteilen erregte Schwingungen sowie zumindest ein zusätzlicher vom Betriebszustand der Maschinen abhängiger Parameter erfasst und in Abhängigkeit von diesem zusätzlich erfassten Parameter mit einem Schwellwert verglichen werden, sowie auf eine Vorrichtung zur Zustandsüberwachung von solchen Maschinen.

Um den Betrieb von Maschinen und Anlagen dauerhaft gewährleisten zu können, ist es notwendig, die einem Verschleiß ausgesetzten Bauteile zu überwachen, um zeitgerecht erforderliche Wartungsarbeiten vornehmen zu können. Dies gilt insbesondere im Bereich von rotierenden Bauteilen, also beispielsweise im Bereich von Lagern oder Getrieberädern. Zu diesem Zweck ist es üblich, die von den rotierenden Bauteilen erregten Schwingungen zu erfassen, die sich mit dem Verschleiß der sie erregenden Bauteile ändern. Damit solche Änderungen im Bereich des zur Zustandsüberwachung erfassten Körperschalls mit größerer Sicherheit einem Verschleiß zugeordnet werden können und nicht auf einer Geometrieänderung zufolge von Wärmedehnungen beruhen, wurde bereits vorgeschlagen (WO 2013/152797 A1), wenigstens einen zusätzlichen vom Betriebszustand der zu überwachenden Maschine abhängigen Parameter, wie die Betriebstemperatur, die Drehzahl oder die Richtung oder Stärke eines Magnetfelds, zu erfassen, sodass aufgrund der Bezugnahme auf diesen zusätzlichen Parameter Fehleinflüsse auf die Zustandsüberwachung zumindest teilweise ausgeschaltet werden können. Trotz der Erfassung dieser zusätzlichen physikalischen Einflussgrößen kann eine zeitliche Voraussage eines wahrscheinlichen Schadenfalls nur in einem vergleichsweise großen Toleranzbereich getroffen werden.

Der Erfindung liegt somit die Aufgabe zugrunde, ein Verfahren zur Zustandsüberwachung von Maschinen mit rotierenden Bauteilen so auszugestalten dass die Voraussetzungen für eine genauere Vorhersage des zeitlichen Eintritts eines Schadenfalls erheblich verbessert werden.

Ausgehend von einem Verfahren der eingangs geschilderten Art löst die Erfindung die gestellte Aufgabe dadurch, dass als zusätzlicher Parameter der Feststoffanteil des hydraulischen Schmiermittels erfasst wird.

Da der Feststoffanteil bzw. die Zunahme des Feststoffanteils im hydraulischen Schmiermittel eine Folge des mechanischen Verschleißes von Lagern oder Zahnflanken ist, kann durch die Erfassung des Feststoffanteils bzw. die daraus ableitbare Zunahme des Feststoffanteils auf Schadensbilder mit einem Materialabtrag rückgeschlossen werden, sodass eine Unterscheidung zwischen Schäden mit und ohne Materialabtrag möglich wird, was eine erhebliche Verbesserung bezüglich der Genauigkeit einer Schadensvorhersage erlaubt, weil beispielsweise zu erwartende Fehler aufgrund einer beginnenden Rissbildung hinsichtlich der zeitlichen Abstimmung der Wartungsmaßnahmen anders als ein zunehmender Verschleiß zu bewerten sind. Da rotierende Bauteile von Maschinen im Allgemeinen durch ein hydraulisches Schmiermittel geschmiert werden, um die Reibungsverhältnisse zu verbessern und damit den Verschleiß herabzusetzen, kann durch eine Überwachung des Feststoffanteils des hydraulischen Schmiermittels die Zustandsüberwachung von Maschinen mit rotierenden Bauteilen merklich verbessert werden.

Bei bekannten Vorrichtungen zur Zustandsüberwachung von Maschinen mit rotierenden Bauteilen werden ein Sensor zur Aufnahme der von den rotierenden Bauteilen erregten Schwingungen und eine an diesen Schwingungssensor angeschlossene Auswerteeinheit vorgesehen, in der die erfassten Schwingungssignale mit einem Schwellwert verglichen werden, um beim Überschreiten dieses Schwellwerts einen bevorstehenden Schaden anzuzeigen. Mit dem Vorsehen eines an die Hydraulikschmierung für die rotierenden Bauteile angeschlossenen Hydraulikmittelsensors zur Bestimmung des Feststoffanteils des Hydraulikmittels können in einfacher Weise die Genauigkeit der Schadensvorhersage verbessert werden, weil mit der Beaufschlagung der Auswerteeinheit durch diesen zusätzlichen Parameter die Voraussetzung geschaffen wird, die zu erwartenden Schäden hinsichtlich der Schadensart zu unterscheiden, was eine genauere Planung der erforderlichen Wartungsarbeiten aufgrund der besseren Kenntnis der Schadensart ermöglicht. Außerdem können auch Schäden von Maschinen mit sehr langsam umlaufenden Bauteilen zuverlässig erkannt werden, die durch eine Analyse des aufgenommenen Schwingungsspektrums nicht ohne weiteres erkannt werden können.

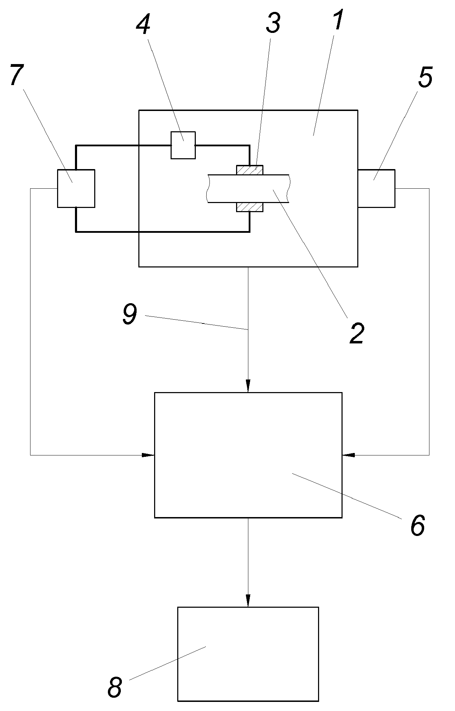

Anhand der Zeichnung wird das erfindungsgemäße Verfahren näher erläutert, und zwar wird eine erfindungsgemäße Vorrichtung zur Zustandsüberwachung von Maschinen in einem schematischen Blockschaltbild gezeigt.

Im dargestellten Blockschaltbild weist die hinsichtlich ihres Zustands zu überwachende Maschine 1 einen rotierenden Bauteil 2, beispielsweise eine Welle auf, deren Lager 3 an eine Hydraulikschmierung 4 angeschlossen sind. Die durch den rotierenden Bauteil 2 angeregten Schwingungen der Maschine 1 werden zumindest bei vorgegebenen Betriebszuständen über einen Sensor 5 erfasst, der an eine Auswerteeinheit 6 angeschlossen ist.

In den Hydraulikkreis der Hydraulikschmierung 4 ist ein Sensor 7 eingebunden, mit dessen Hilfe der Feststoffanteil des Hydraulikmittels in an sich bekannter Weise bestimmt werden kann. Mithilfe der Ausgangssignale des Sensors 7 wird die Auswerteeinheit 6 beaufschlagt, sodass programmbedingt die durch den Sensor 5 erfassten Schwingungen in Abhängigkeit vom jeweils ermittelten Feststoffanteil des hydraulischen Schmiermittels bzw. von der daraus abgeleiteten Zunahme des Feststoffanteils mit einem entsprechenden Schwellwert verglichen werden kann, um beim Überschreiten des Schwellwerts einen bevorstehenden Lagerschaden anzuzeigen. Zu diesem Zweck ist die Auswerteeinheit 6 mit einer Anzeigeeinheit 8 verbunden.

Da insbesondere das Schwingungsverhalten der Maschine 1 von deren Betriebszustand abhängt, werden die über den Sensor 5 erfassten Schwingungen mit dem jeweiligen Betriebszustand der Maschine 1 in Beziehung gesetzt. Der jeweilige Betriebszustand der Maschine 1 wird der Auswerteeinheit 6 über eine Leitung 9 übermittelt. Zeigt eine auf den jeweiligen Betriebszustand bezogene Schwingungsanalyse eine Überschreitung eines für diesen Betriebszustand vorgegebenen Schwellwerts an, so kann aufgrund der zusätzlichen Information über den Feststoffanteil im hydraulischen Schmiermittel beurteilt werden, ob der angezeigte Schaden auf einen Materialabtrag oder eine Unwucht oder beginnende Rissbildung ohne Materialabtrag zurückzuführen ist. Selbstverständlich kann auch eine Kombination dieser Schadensarten vorliegen und angezeigt werden, sodass aufgrund der Möglichkeit, Rückschlüsse auf die Schadensart zu ziehen, die notwendigen Wartungsarbeiten genauer geplant werden können.

## Patentansprüche

1. Verfahren zur Zustandsüberwachung von Maschinen (1) mit rotierenden, durch ein hydraulisches Schmiermittel geschmierten Bauteilen (2), wobei von den rotierenden Bauteilen (2) erregte Schwingungen sowie zumindest ein zusätzlicher vom Betriebszustand der Maschinen (1) abhängiger Parameter erfasst und in Abhängigkeit von diesem zusätzlich erfassten Parameter mit einem Schwellwert verglichen werden, **dadurch gekennzeichnet, dass** als zusätzlicher Parameter der Feststoffanteil des hydraulischen Schmiermittels erfasst wird.

2. Vorrichtung zur Zustandsüberwachung von Maschinen (1) mit rotierenden Bauteilen (2), mit einer Hydraulikschmierung (4) für die rotierenden Bauteile (2), mit zumindest zwei Sensoren (5, 7) einerseits für von den rotierenden Bauteilen (2) erregte Schwingungen und anderseits für einen vom Betriebszustand der Maschinen (1) abhängiger zusätzlichen Parameter sowie mit einer an die Sensoren (5, 7) angeschlossenen Auswerteeinheit (6) zum Vergleich der Signale des Schwingungssensors mit einem Schwellwert in Abhängigkeit von den Signalen des Sensors (7) für den zusätzlichen Parameter, **dadurch gekennzeichnet, dass** der Sensor (7) für den zusätzlichen Parameter ein an die Hydraulikschmierung (4) angeschlossener Hydraulikmittelsensor zur Bestimmung des Feststoffanteils des Hydraulikmittels ist.
